# EUROPÄISCHE PATENTSCHRIFT

(11) **EP 1 414 958 B1**
(45) Veröffentlichungstag und Bekanntmachung des Hinweises auf die Patenterteilung: **29.08.2007**
(21) Anmeldenummer: 02764527.4
(22) Anmeldetag: 17.07.2002
(51) Int. Cl.: C12N 15/10, C12P 21/00

(54) **VERFAHREN ZUR GENEXPRESSION**
METHOD FOR GENE EXPRESSION
PROCEDE D'EXPRESSION GENETIQUE

(30) Priorität: 06.08.2001 DE 10137792
(43) Veröffentlichungstag der Anmeldung: 06.05.2004
(73) Patentinhaber: Erdmann, Volker A., 14163 Berlin (DE)
(72) Erfinder: ERDMANN, Volker, A., 14163 Berlin (DE); LAMLA, Thorsten c/o Boehringer Ingelheim Pharma, 88397 Biberach/Riss (DE); STIEGE, Wolfgang, 14195 Berlin (DE)
(74) Vertreter: Jungblut, Bernhard Jakob
(86) Internationale Anmeldenummer: PCT/DE2002/002672
(87) Internationale Veröffentlichungsnummer: WO 2003/016526

(56) Entgegenhaltungen:
- EP-A- 0 312 617
- EP-A- 0 314 415
- EP-A- 0 593 757
- EP-A- 1 176 210
- US-A- 5 593 856
- LAMLA THORSTEN ET AL: "An improved protein bioreactor: Efficient product isolation during in vitro protein biosynthesis via affinity tag." MOLECULAR & CELLULAR PROTEOMICS, Bd. 1, Nr. 6, Juni 2002 (2002-06), Seiten 466-471, XP002223429 June, 2002 ISSN: 1535-9476

## Beschreibung

### Gebiet der Erfindung

Die Erfindung betrifft ein Verfahren zur Genexpression in einem zellfreien Transkriptions/Translations-System gemäß dem Oberbegriff des Anspruchs 1.

Weiterhin betrifft die Erfindung eine Vorrichtung zur zellfreien Genexpression in einem Transkriptions/Translations-System.

### Stand der Technik

Verfahren zur zellfreien Expression von Proteinen sind beispielsweise aus den Literaturstellen EP 0312 617 B1, EP 0401 369 B1 und EP 0 593 757 B1 bekannt.

Demgemäß werden die für eine Transkription und/oder Translation notwendigen Komponenten neben einem für ein gewünschtes Protein kodierenden Nukleinsäurestrang in einem Reaktionsgefäß inkubiert und nach der Expression die Polypeptide/Proteine aus der Reaktionslösung isoliert.

Sowohl die für die Transkription, als auch die für die Translation notwendigen Komponenten lassen sich leicht aus den Überstanden pro- oder eukaryontischer Zelllysate nach beispielsweise einer 30000 x g Zentrifugation gewinnen. Dieser sogenannte S-30 Extrakt enthält alle die für die Transkription und Translation notwendigen Komponenten.

In den meisten Fällen befindet sich das Gen, bzw. der für das Protein oder Peptid kodierende Nukleinsäurestrang, unter der Kontrolle eines T7-Promotors. Dies hat zum Vorteil, daß durch den Einsatz von Rifampicin vorhandene *E.coli* RNA-Polymerase inhibiert werden kann, und somit etwaige aus dem S30-Extrakt oder aus der Vektorpräparation stammende endogene *E.coli* DNA nicht transkribiert wird. Wird dagegen ein unter einem *E.coli* Promotor stehendes Gen exprimiert, muß eine *E.coli* Polymerase eingesetzt werden, was zu einer Co-expression etwaiger endogener *E.coli* DNA und somit zu unerwünschten, endogenen Proteinen führen kann. Die Expression erfolgt typischerweise bei 37°C, kann aber auch bei Temperaturen von 17°C bis 45°C erfolgen. Die Anpassung der Temperatur empfiehlt sich insbesondere bei der Expression von Proteinen, in denen eine komplexe Sekundär-/Tertiärstruktur ausgebildet werden soll. Durch Absenken der Temperatur kann die Syntheserate gesenkt und somit den Proteinen die Möglichkeit gegeben werden, sich richtig zu falten, um ein funktionstüchtiges/aktives Protein zu erhalten. Auch kann auf die Ausbildung von Disulfidbrücken innerhalb der exprimierten Proteine Einfluß über das Reduktionspotetial der Reaktionslösung durch den Zusatz von Dithiothreitol (DTT) und/oder oxidiertem/reduziertem Glutathion genommen werden.

Vor jeder neuen Proteinsynthese müssen die jeweiligen Systeme einer Optimierung unterzogen werden. Dabei werden die Konzentrationen der zweiwertigen Magnesium-Ionen (Mg²⁺), der RNA/DNA-Polymerase und des als Matrize dienenden kodierenden Nukleinsäurestrangs variiert.

In dem im Dokument EP 0312 617 B1 offenbarten Verfahren zur zellfreien Expression von Proteinen wird der für das Protein kodierende Nukleinsäurestrang als mRNA der Reaktionslösung zugegeben. Damit müssen für die Herstellung von Polypeptiden in dem zellfreien System nur die für die Translation notwendigen Komponenten des Translationsapparates, insbesondere Ribosomen, Initiations-, Elongations-, Freisetzungsfaktoren und Aminoacyl-tRNA-Synthetase, sowie Aminosäuren und als Energie liefernde Substanzen ATP und GTP in ein Reaktionsgefäß gegeben werden. Bei der anschließenden Polypeptid-/Proteinsynthese kommt es neben der Bildung von Polypeptiden/Proteinen auch zur Bildung von niedermolekularen Substanzen, wie ADP, AMP, GDP, GMP und anorganischen Phosphaten unter Verbrauch der Energie liefernden Substanzen ATP und GTP und von Aminosäuren. Diese führt dazu, daß die Reaktion nach dem Verbrauch von ATP oder GTP oder einer Aminosäure, bzw. durch die als Inhibitoren wirkenden gebildeten niedermolekularen Substanzen nach etwa 30 bis 60 Minuten zum Erliegen kommt. Um dem entgegen zu wirken, offenbart die Literaturstelle EP 0312 617 B1 daß die während der Translation verbrauchten Substanzen während der Translation herausgeführt und gleichzeitig die Energie liefernden Substanzen und die Aminosäuren zur Erhaltung der Ausgangskonzentration eingeführt werden.

Demgegenüber offenbart das Dokument EP 0401 369 B1 ein Verfahren, bei dem die für das Protein kodierende Nukleinsäurestrang als mRNA oder DNA der Reaktionslösung zugegeben werden kann. Letzteres hat den Vorteil, daß DNA wesentlich stabiler als mRNA ist, und der notwendige Umschreibeprozess der DNA in RNA vor der Reaktion nicht notwendig ist, sondern gleich die DNA, z.B. als Vektor oder lineares Konstrukt eingesetzt werden kann. Durch den Einsatz der DNA muß das zellfreie Expressionssystem neben den obengenannten Translationsfaktoren noch die für die Transkription der DNA in RNA notwendigen Transkriptionsfaktoren, wie z.B. RNA-Polymerase, -Faktor und Rho-Protein und die Nukleotide ATP, UTP, GTP und CTP enthalten. Auch hier müssen die während der Transkription/Translation verbrauchten niedermolekularen Substanzen, wie ADP, AMP, GDP, GMP und anorganischen Phosphate während der Translation herausgeführt werden und gleichzeitig die Energie liefernde Substanzen, Nukleotide und die Aminosäuren zur Erhaltung der Ausgangskonzentration eingeführt werden.

Beide insofern bekannte Verfahren haben den Nachteil, daß die translatierten Proteine während der gesamten Reaktionszeit in dem Reaktionsgefäß in Lösung bleiben, und erst nach der Reaktion durch geeignete Reinigungsmethoden aus der Reaktionslösung entfernt werden können. Dies führt dazu, daß der Proteinexpression ein zeitaufwendiger separater Verfahrensschritt anzuschliessen ist, in dem es den Proteinen ermöglicht werden muß, an eine Matrix oder ähnliches zu binden, um es von den restlichen Bestandteilen der Reaktionslösung zu trennen.

Ein weiteres Problem liegt darin, daß während der Expression, die bis zu 100 Stunden dauern kann, ohne Probenentnahme keinerlei Informationen über die Menge und/oder Aktivität an erhaltenem Protein erhalten werden. Durch eine Probenentnahme würde aber wiederum ein Teil der Lösung dem Reaktionssytem entzogen, was zu einer geringeren Gesamtausbeute führt. Die Kontrolle der Proteinsynthese ist insbesondere deswegen notwendig, weil durch den Mangel von nur einer einzigen der vielen für die Transkription/Translation notwendigen Komponenten die Syntheserate drastisch verringert oder gar zum Erliegen kommen kann. Auch können die exprimierten Proteine bei höheren Konzentrationen ausfallen oder ihre eigenen Synthese als Produkt hemmen.

Aus dem Dokument EP 0 593 757 B1 ist es bekannt, neben den verbrauchten niedermolekularen Substanzen auch die exprimierten Polypeptide durch eine Ultrafiltrationsbarriere während der Translation aus der Reaktionslösung abzutrennen. Diese Abtrennung der Polypeptide erfolgt somit nur allein über die Porengröße der eingesetzten Ultrafiltrationsbarriere/-membran und hat zum Nachteil, daß man in der Wahl der Porengröße stark limitiert ist, da die Porengröße nicht so groß werden darf, daß die für die Transkription/Translation notwendigen Faktoren und Enzyme die Membran passieren können. Somit können während der Expression nur kleinere Polypeptide und keine größeren Polypeptide oder gar Proteine abgetrennt werden. Auch kann es zu Interaktionen zwischen Proteinen und der Ultrafiltrationsbarriere/-membran kommen, was die Proteinausbeute verringern kann.

Aus der Literaturstelle US 5,593,856 A ist ein Verfahren zur Genexpression in einem zellfreien Transkriptions/Translationssystem bekannt, wobei die entstehenden Proteine durch Zuleitung der Reaktionslösung in einer Protein-isolierenden Vorrichtung, beispielsweise einer Säule mit einer Matrix mit Antikörpern, welche das entstehende Protein binden, isoliert werden.

### Technisches Problem der Erfindung

Der Erfindung liegt das technische Problem zugrunde, ein Verfahren zur Genexpression in einem zellfreien Transkriptions/Translations-System zu entwickeln, wobei eine Abtrennung von Produkt-Protein einfach und ohne separaten Isolierungsschritt erfolgen kann und wobei die Menge an exprimierten Polypeptid/Protein vor dem Ende der Reaktion bestimmt werden kann. Insbesondere sollen die während der Reaktion erhaltenen Proteine unabhängig von ihrer Größe der Reaktionslösung abtrennbar sein.

Grundzüge der Erfindung und bevorzugte Ausführungsformen.

Zur Lösung dieses technischen Problems lehrt die Erfindung das Merkmal gemäß dem Kennzeichnungsteil des Anspruchs 1. Im Rahmen des Verfahrens wird nicht mit zwei Verfahrensstufen, Transkription/Translation und anschließenden Produktabtrennung, gearbeitet, sondern die Immobilisierung wird bereits mit Beginn der Transkription/Translation durchgeführt und hält während der Transkription/Translation an, erfolgt also parallel zu der Genexpression. Zum Ende der Reaktion wird das Immobilisat entnommen und das immobilisierte Protein wieder abglöst.

Mit der Erfindung wird eine beachtliche Vereinfachung der Verfahrensabläufe erreicht. Zudem wird das Produkt-Protein ständig entzogen, so daß dieses die Transkription/Translation nicht stören kann. Hierdurch werden auch Proteine der Synthese zugänglich, die ansonsten allenfalls sehr geringe Ausbeuten wegen negativer Wechselwirkungen mit dem System liefern.

Diese Immobilisierung erfolgt außerhalb der Reaktionskammer.

Die Reaktionslösung wird während der Reaktion aus der Reaktionskammer heraus über die Matrix und wieder in die Reaktionskammer zurück geleitet. Hierbei befindet sich die Matrix selber in einem separaten Reaktionsgefäß.

Durch eine Proteinbestimmung vor und nach dem separaten Reaktionsgefäß (z.B. eine Säule), kann über die Differenz der Proteinmengen die absorbierte Proteinmenge bestimmt werden und für den Fall, daß die Matrix spezifisch das exprimierte Protein bindet, auch direkt die exprimierte Proteinmenge und über den zeitlichen Verlauf auch die Expressions-/Syntheserate bestimmt werden. In einfachsten Fall, erfolgt die Bestimmung der Proteinkonzentration durch eine Messung der Absorption der Reaktionslösung bei der Wellenlänge 1 bis 280 nm. Mit Hilfe des Extinktionskoeffizienten kann über das Lambert-Beer'sche Gesetz die Proteinmenge der Lösung bestimmt werden. Um die Proteinkonzentration möglichst genau zu ermitteln, ist es durchaus sinnvoll, neben der Proteinkonzentration auch die Nukleotidkonzentration in der Reaktionslösung zu bestimmen. Dies kann z.B. durch Messung der Absorption bei 2-260 nm erfolgen, die eine Aussage über den Gehalt an Nukleotiden in der Lösung ermöglicht. Damit können Fehler bei der Proteinbestimmung, insbesondere durch Messung der Absorption, verhindert werden, da hohe Nukleotidkonzentrationen die Proteinmessung beeinflussen können.

Auch ist man in der Lage, schon während der Inkubationszeit nicht nur das Protein zu binden, sondern auch das gebundene Protein von der Matrix zu isolieren. Hierzu wird die in einem separaten Reaktionsgefäß/Säule befindliche Matrix von dem Kreislauf abgekoppelt, wobei der Kreislauf dann z.B. auf eine parallel geschaltete Matrix umgelenkt werden kann. Gegenüber der im Dokument EP 583757 B1 beschriebenen Abtrennung hat die Erfindung zum Vorteil, daß die an die Matrix immobilisierten Proteine schon konzentriert sind und daß somit nur ein sehr geringer Teil der Reaktionslösung aus dem System entfernt werden muß. Auch ist die Abtrennung im Gegensatz zu dem in dem Dokument EP 583757 B1 offenbarten Verfahren unabhängig von der Größe der Proteine, da sie nicht aufgrund ihrer Größe aus der Reaktionslösung abgetrennt werden, sondern spezifisch an einer Matrix immobilisiert werden. Somit können auch Proteine der Reaktionslösung entzogen werden, die z.B. so groß sind, wie die in der Lösung befindlichen, für Transkription/Translation notwendigen Proteine. Es ist somit möglich, die niedermolekularen Stoffwechselprodukte, die eine Größe von weniger als 10 kD haben, durch eine Membran abzutrennen, und gleichzeitig die Proteine, die für die Transkription/Translation notwendig sind und typischerweise größer als 50 kD sind, in der Lösung zu behalten und gleichzeitig die exprimierten Proteine unabhängig von ihrer Größe an der Matrix zu binden.

Erfindungsgemäß wird so vorgegangen, dass die Proteinkonzentration vor und nach der Säule nach dem oben beschriebenen Verfahren bestimmt wird, um die optimale Beladung der Säule zu erzielen, und somit den Verlust an Reaktionslösung zu minimieren. Eine optimale Beladung der Matrix ist dann erzielt, wenn die vor und nach der Säule gemessene Proteinkonzentrationen identisch sind, und somit kein Protein mehr an die Säule bindet.

Die Entnahme der Proteine schon vor dem Reaktionsende ist insbesondere dann empfehlenswert, wenn es sich bei den Proteinen um Proteine handelt, deren Aktivität wesentlich ist, wie z.B. bei Kinasen der Fall. Hierbei stellt eine Feststellung fehlender Aktivität ein Abbruchkriterium für das Verfahren dar, und zwar bereits nach einer Zeitdauer, die einen kleinen Bruchteil der Reaktionsgesamtdauer beträgt, beispielsweise 1/10 oder 1/20 oder weniger. Durch die Entnahme ist man also schon während des Prozesses in der Lage, auch die Qualität der Proteine zu beurteilen, und zwar ohne dabei nennenswerte Verluste an Reaktionslösung in Kauf zu nehmen.

Schließlich kann durch die Immobilisierung verhindert werden, daß die exprimierten Proteine bei höheren Konzentrationen ausfallen, oder ihre eigenen Synthese als Produkt hemmen.

Die Funktionsweise der Matrix kann auf allen für die Bindung von Proteinen bekannten Reinigungsmethoden, wie z.B. Ionentauscher, Affinität, Antigen/Antikörper-Wechselwirkung, hydrophobe/hydrophile-Wechselwirkung beruhen.

Für eine besonders effiziente Reinigung der Proteine können diese mit einem oder mehreren Markern, z.B. in Form von mehreren N- oder C-terminalen aufeinander folgenden Histidinen, oder einem oder mehreren anderen Protein, z.B. Glutathion, als ein sogenanntes Fusionsprotein Ko-Exprimiert werden. Die Matrix weist dann ein für diesen Marker/dieses Protein einen spezifische Bindungspartner auf, die eine effiziente Bindung des chimären Fusionsproteins über den Marker/den Fusionpartner an die Matrix ermöglichen.

Um eine kontinuierliche Proteinexpression zu gewährleisten, können die während der Transkription/Translation verbrauchten Substanzen während der Transkription/Translation herausgeführt werden und gleichzeitig die Energie liefernde Substanzen und die Aminosäuren zur Erhaltung der Ausgangskonzentration über eine semipermeable Membran eingeführt werden. Des weiteren können die verbrauchten niedermolekularen Substanzen durch eine Ultrafiltrationsmembran während der Transkription/Translation aus der Reaktionslösung abgetrennt werden, während gleichzeitig über eine Pumpe die Energie liefernde Substanzen und die Aminosäuren zur Erhaltung der Ausgangskonzentration über eine weitere Pumpe zugeführt werden.

Die Erfindung kann grundsätzlich sowohl in prokaryontischen Systemen als auch in eukaryontischen Systemen eingesetzt werden.

Bevorzugt ist es hinsichtlich des Verfahrens, daß sich die Matrix außerhalb der Reaktionskammer, vorzugsweise in einem weiteren Reaktionsgefäß, höchtvorzugsweise in einer Säule, befindet. Wesentlich ist es, daß vor und nach der Matrix die Proteinkonzentration bestimmt wird, vorzugsweise durch Absoption von Licht, insbesondere im UV-Bereich. Die Matrix kann aufgrund von hydrophoben- und/oder hydrophilen oder Antigen/Antikörper oder ionischen Wechselwirkungen spezifisch Proteine binden. Hierbei sind die geeigneten Moleküle an der Oberfläche eines Substrates gebunden.

Die Matrix kann Anionen- oder Kationenaustauschermaterial oder Hydroxyapatid enthalten. Wenn die Proteine als Fusionsproteine exprimiert werden und die Fusionspartner N-, C-terminal oder innerhalb des exprimierten Proteins liegen, kann eine Matrix verwendet werden, die spezifisch den Fusionspartner bindet. So kann das Protein N- oder C-terminal mehrere aufeinander folgende Histidine, insbesondere 3 bis 12, vorzugsweise 5 bis 9, höchst vorzugsweise 6, enthalten, wobei die Matrix dann eine Metall-Chelat-Verbindung tagen kann, insbesondere mit zweiwertigen MetallIonen, vorzugsweise zweiwertige Kupfer- und/oder Nickel-Ionen. Das Protein kann N- oder C-terminal als Fusionspartner Gluthtion-S-Transferase enthalten, wobei dann an die Matrix Glutathion gekoppelt sein kann. Das Protein kann ein Aminosäuresequenz enthalten, die es ihm ermöglicht an Streptavidin zu binden, vorzugsweise die Aminosäuresequenz AWRHPQFGG, höchstvorzugsweise die Aminosäuresequenz WSHPQFEK, wobei dann an die Matrix Streptavidin gekoppelt sein kann.

Entstehende niedermolekulare Stoffwechselprodukte können während der Expression abgeführt und verbrauchte notwendige Stoffwechselbestandteile der Reaktion in dem Maße zugeführt werden, daß die Anfangskonzentrationen der niedermolekulare Stoffwechselprodukte und notwendige Stoffwechselbestandteile konstant bleiben, wobei die notwendigen Stoffwechselbestandteile der Reaktion aus der nicht abschließenden Gruppe "ATP, UTP, GTP und CTP, Pyrophosphat und Aminosäuren" gebildet werden und, wobei die niedermolekulare Stoffwechselprodukte aus der nicht abschließenden Gruppe "ADP, AMP, GDP, GMP und anorganischem Phosphat" gebildet werden. Die notwendigen Stoffwechselbestandteile können über eine semipermeable Membran zu- und/oder die niedermolekulare Stoffwechselprodukte abgeführt werden.

Im Folgenden wird die Erfindung anhand von lediglich Ausführungsformen darstellenden Figuren sowie einem Beispiel näher erläutert. Es zeigen:

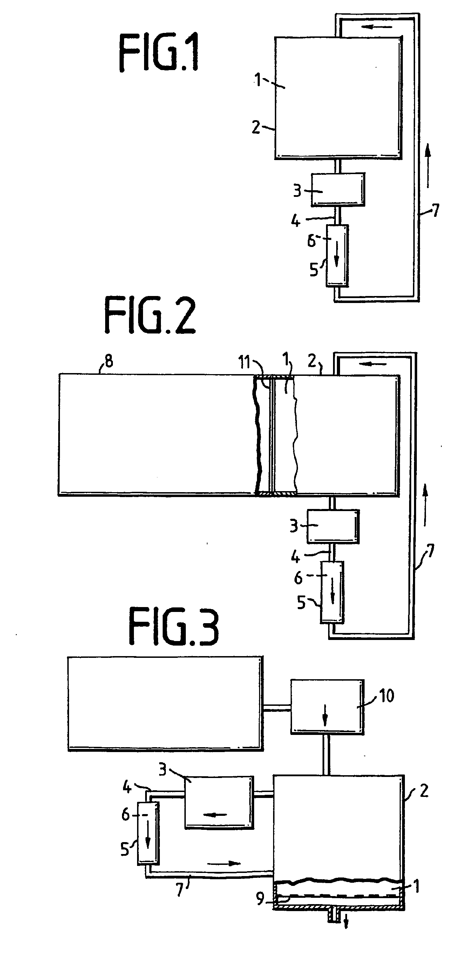

- Fig. 1:: eine schematische Darstellung einer erfindungsgemäßen Vorrichtung mit einer Reaktionskammer, einer die Matrix enthaltenden Säule und einer Pumpe.
- Fig. 2:: eine schematische Darstellung einer erfindungsgemäßen Vorrichtung mit einer Reaktionskammer, einer die Matrix enthaltenden Säule und einer Pumpe, bei der die notwendigen Stoffwechselbestandteile über eine semipermeable Membran zu- und die niedermolekulare Stoffwechselprodukte abgeführt werden (semi-kontinuierlicher zellfreier Reaktor).
- Fig. 3:: eine schematische Darstellung einer erfindungsgemäßen Vorrichtung mit einer Reaktionskammer, einer die Matrix enthaltenden Säule und einer Pumpe, bei der die niedermolekulare Stoffwechselprodukte über eine Ultrafiltrationsmembran abgeführt werden (kontinuierlicher zellfreier Reaktor).

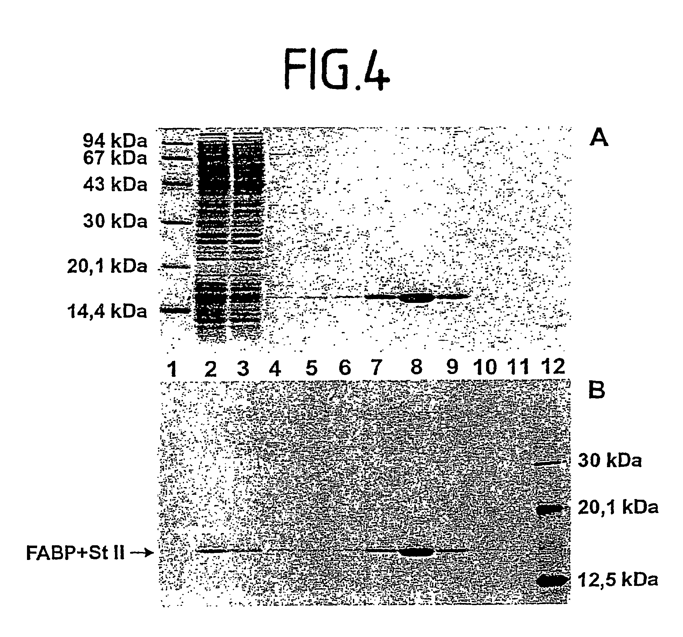

- Fig. 4:: Isolierung des FABP mit Strep-tag II aus dem semi-kontinuierlichen zellfreien Reaktor während der Synthese mit einer StrepTactin Sepharose Säule. Aufgetragen wurden von jeder isolierten Fraktion 15 µl und analysiert mit SDS-PAGE unter reduzierenden Bedingungen. Aufgetragen wurden die Proben wie folgt: (1) Molekulargewichtsmarker; (2) Reaktionslösung; (3 bis 5) Waschfraktionen 1 bis 3; (6 bis 11) eluierte Fraktionen 1 bis 6 und (12) L[14C]-Leucin Molekulargewichtsmarker. (A) Coomassie gefärbtes SDS-PAGE und (B) Autoradiogram des SDS-PAGE.

In der Figur 1 erkennt man eine die Reaktionslösung 1 aufnehmende Reaktionskammer 2, in der die Transkription/Translation erfolgt. Über eine Pumpe 3 wird über eine Zuleitung 4 die Reaktionslösung 1 über die sich in einem separaten Reaktionsgefäß, nämlich einer Säule 5, befindliche Matrix 6 und über eine Ableitung 7 zurück in das Reaktionsgefäß 2 gepumpt.

Die Figur 2 zeigt eine gegenüber dem Gegenstand der Figur 1 ergänzte erfindungsgemäße Vorrichtung, wobei die Reaktionskammer 2 über eine semipermeable Membran mit einem Vorlagegefäß 8 verbunden ist. Über die semipermeable Membran 11 werden die während der Transkription/Translation verbrauchten Substanzen während der Transkription/Translation herausgeführt und gleichzeitig die Energie liefernde Substanzen und die Aminosäuren zur Erhaltung der Ausgangskonzentration zugeführt.

Die Figur 3 zeigt eine gegenüber dem Gegenstand der Figur 1 ergänzte erfindungsgemäße Vorrichtung, wobei die verbrauchten niedermolekularen Substanzen durch eine Ultrafiltrationsmembran 9 während der Transkription/Translation aus der Reaktionslösung 1 abgetrennt werden können, während gleichzeitig über eine weitere Pumpe 10 die Energie liefernden Substanzen und die Aminosäuren zur Erhaltung der Ausgangskonzentration aus einem Vorlagegefäß 8 zugeführt werden können.

### Beispiel:

Eine gekoppelte Transkription/Translation wurde in einem semi-kontinuierlichen zellfreien Reaktor gemäß der Figur 2 für 20 Stunden bei 30°C durchgeführt. Die Reaktionskammer 2 hatte ein Volumen vom 750 µl, und in Verbindung mit den Volumina der Pumpe 3 der Zuleitung 4, Ableitung 7 und der Säule ergab sich ein Gesamtvolumen der Reaktionslösung 1 von 2150 µl. Die Reaktionslösung für das gekoppelte Transkriptions-/Tanslationssystem basiert auf einem *E. coli* S30 Lsysat (Stamm D10). Das eingesetzte Plasmid war pHMFH + StII. Ausgangsvektor für dieses Plasmid war pUC18. Hierein wurde das SphI/EcoRI-Fragment aus pET3d (Novagen) kloniert. In diesen veränderten Vektor wurde das FABP-Gen (Fatty acid binding protein) über NcoI/BamHI kloniert. Das eingesetzte Transkriptions-/Translationssystem ist beschrieben von Zubay, G. (Annu. Rev. Genet. 7, 267-287 und wurde komplettiert mit 500 U/ml T7 Phagen RNA Polymerase (Stratagen) and 200 µM L[14C]-Leucin (25 dpm/pmol, erhältlich aus 150 µM "kaltem" und 50 µM "heißem" Leucin, Amersham, 100 dpm/pmol). Der für FABP kodierende Nukleinsäurestrang wurde in Form des vorstehenden Plasmids dem System in einer Konzentration von 2 nM zugesetzt, in dem das FABP mit einem C-terminalen Strep-tag II exprimiert wird.

In das Vorlagegefäß wurden 6 ml Translationspuffer (50 mM HEPES-KOH, (pH 7,6), 70 mM KOAc, 30 mM NH4Cl, 10 mM MgCl2, 0,1 mM EDTA (ph 8,0), 0,002% NaN3) mit 5 mM Dithiothritol, 4 mM MgC12, 0,02% NaN3; 100 µM Folsäure, 400µM L[14C]-Leucin (0,75 dpm/pmol, erhältlich aus 397µM kaltem und 3pM heißem Leucin; Amersham, 100 dpm/pmol), 400µM von jeder der anderen 19 Aminosäuren, je 1 mM von ATP und GTP, je 0,5 mM von CTP und UTP, 30 mM Phosphoenolpyruvat (Boehringer Mannheim), 10 nM Acetylphosphat (Sigma) gegeben. Während der Synthese wurde die Reaktionslösung 1 kontinuierlich mit einer Flußrate von circa 0,07 ml/min mit Hilfe der Pumpe 3 von der Reaktionskammer 2 über die Zuleitung 4 auf die in der Säule 5 befindliche Matrix 6 und über die Ableitung 7 wieder in die Reaktionskammer 2 im Kreis gepumpt. Als Matrix dienten 530 µl StrepTactin Sepharose (IBA Göttingen).

Zur Isolierung des gebundenen Produktes wurden die StrepTactin Sepharose drei mal mit 800 µl Waschpuffer (100 mM Tris-HCl (pH 8,0), 150 mM NaCl, 1 mM EDTA) und das Fusionsprotein sechs mal mit Elutionspuffer (50 mM HEPES-KOH, (pH 7,6), 70 mM KOAc, 30 mM NH4Cl, 10 mM MgCl2, 0,1 mM EDTA (pH) 8,0), 0,002% NaN3, 2,5 mM Desthiobiotin) eluiert.

Die Messung der Inkorporation des L[14C]-Leucin in das synthetisierte Protein erfolgte durch Szintillationszählung von mit Trichloressigsäure gefälltem Material, wie beschrieben von Merk, H.; Stiege, W., Tsumoto, K. Kumagai, I. und Erdmann, V.A. (1999, J. Biochem. 125, 328-333). Es konnte gezeigt werden, daß 41% des exprimierten Proteins in der Reaktionslösung verblieben war, 13% waren in der mit dem Waschpuffer eluierten Fraktion und 46% des exprimierten Proteins konnten von der Säule eluiert werden. Die exprimierten Proteine wurden des Weiteren in der Polyacrylamidgelelektrophorese (SDS-PAGE) unter denaturierten Bedingungen aufgetrennt und durch Coomassie-Färbung und mit Hilfe des Phosphorimager System (Molecular Dynamics) analysiert. Die oben aufgeführte Verteilung der exprimierten Proteine auf die einzelnen Fraktionen konnte sowohl im Coomassie (Fig. 4 A) gefärbten SDS-PAGE, als auch im entsprechenden Autoradiogram (Fig. 4 B) bestätigt werden. Durch eine Vergrößerung der Matrix konnte eine eindeutige Verschiebung des an die Matrix gebundenen Proteins erzielt werden.

## Patentansprüche

1. Verfahren zur Genexpression in einem zellfreien Transkriptions/Translations-System, wobei die Reaktionslösung alle notwendigen Komponenten des Transkriptions-/Translationsapparates, Aminosäuren, Nukleotide, Energie liefernden und für die Synthese notwendigen Stoffwechselbestandteile enthält,
wobei entstehende Proteine während der Transkription/Translation **dadurch** an einer Matrix immobilisiert werden, dass die Reaktionslösung über die außerhalb einer Reaktionskammer, die zur Aufnahme der Reaktionslösung dient, in einem von der Reaktionslösung durchströmten separaten Reaktionsgefäß angeordnete Matrix geleitet wird,
**dadurch gekennzeichnet,**
**daß**, bezogen auf die Strömungsrichtung der Reaktionslösung, vor und nach der Matrix die Proteinkonzentration bestimmt wird.

2. Verfahren nach Anspruch 1, **dadurch gekennzeichnet**, das die Matrix in einer Säule angeordnet ist.

3. Verfahren nach Anspruch 1 oder 2, **dadurch gekennzeichnet, dass** die Bestimmung der Proteinkonzentration durch Absorption von Licht, insbesondere im UV-Bereich, durchgeführt wird.

4. Verfahren nach einem der Ansprüche 1 bis 3, **dadurch gekennzeichnet, daß** die Matrix aufgrund von hydrophoben- und/oder hydrophilen oder Antigen/Antikörper oder ionischen Wechselwirkungen spezifisch Proteine bindet.

5. Verfahren nach einem der Ansprüche 1 bis 4, **dadurch gekennzeichnet, daß** die Matrix Anionen- oder Kationenaustauschermaterial oder Hydroxyapatid enthält.

6. Verfahren nach einem der Ansprüche 1 bis 5, **dadurch gekennzeichnet, daß** die Proteine als Fusionsproteine enthaltend einen Fusionspartner exprimiert werden.

7. Verfahren nach Anspruch 6, **dadurch gekennzeichnet, daß** die Fusionspartner N-terminal, C-terminal oder innerhalb des exprimierten Proteins liegen.

8. Verfahren nach Anspruch 6 oder 7, **dadurch gekennzeichnet, daß** eine Matrix verwendet wird, die spezifisch den Fusionspartner bindet.

9. Verfahren nach einem der Ansprüche 6 bis 8, **dadurch gekennzeichnet, daß** das Protein N-terminal oder C-terminal mehrere aufeinander folgende Histidine, insbesondere 3 bis 12, vorzugsweise 5 bis 9, höchst vorzugsweise 6, enthält.

10. Verfahren nach einem der Ansprüche 1 bis 9, **dadurch gekennzeichnet, daß** die Matrix eine Metall-Chelat-Verbindung trägt, insbesondere mit zweiwertigen Metall-Ionen, vorzugsweise zweiwertige Kupfer- und/oder Nickel-Ionen.

11. Verfahren nach einem der Ansprüche 6 bis 8, **dadurch gekennzeichnet, daß** das Protein N- terminal oder C-terminal als Fusionspartner Gluthation-S-Transferase enthält.

12. Verfahren nach einem der Ansprüche 1 bis 11, **dadurch gekennzeichnet, daß** an die Matrix Glutathion gekoppelt ist.

13. Verfahren nach einem der Ansprüche 6 bis 8, **dadurch gekennzeichnet, daß** das Protein ein Aminosäuresequenz enthält, die es ihm ermöglicht, an Streptavidin zu binden.

14. Verfahren nach einem der Ansprüche 1 bis 13, **dadurch gekennzeichnet, daß** an die Matrix Streptavidin gekoppelt ist.

15. Verfahren nach einem der Ansprüche 1 bis 14, **dadurch gekennzeichnet, daß** entstehende niedermolekulare Stoffwechselprodukte während der Expression abgeführt und verbrauchte notwendige Stoffwechselbestandteile der Reaktionkammer in dem Maße zugeführt werden, daß die Konzentrationen der niedermolekulare Stoffwechselprodukte und notwendige Stoffwechselbestandteile während der Reaktionsdauer um weniger als 80%, bezogen auf Mole einer respektiven Anfangskonzentration, vorzugsweise um weniger als 50%, höchstvorzugsweise um weniger als 20%, von den respektiven Anfangskonzentrationen abweichen.

16. Verfahren nach einem der Ansprüche 1 bis 15, **dadurch gekennzeichnet, dass** die notwendigen Stoffwechselbestandteile einen Stoff der Gruppe bestehend aus "ATP, UTP, GTP und CTP, Pyrophosphat, Aminosäuren und Mischungen dieser Stoffe" enthalten.

17. Verfahren nach einem der Ansprüche 1 bis 16, **dadurch gekennzeichnet, daß** die notwendigen Stoffwechselbestandteile über eine semipermeable Membran zu- und die niedermolekulare Stoffwechselprodukte über eine semipermeable Membran, vorzugsweise die gleiche semipermeable Membran, abgeführt werden, wobei die semipermeable Membran vorzugsweise eine Ausschlußgrenze im Bereich von 500 bis 20000 Da, höchstvorzugsweise im Bereich 3000 bis 5000 Da, aufweist.

18. Vorrichtung zur Durchführung eines Verfahrens nach einem der Ansprüche 1 bis 17, mit einer Reaktionskammer zur Aufnahme der Reaktionslösung, die alle notwendigen Komponenten des Transkriptions-/Translationsapparates, Aminosäuren, Nukleotide, Energie liefernde und für die Synthese notwendige Stoffwechselbestandteile enthält, und mit einer Matrix, die spezifisch exprimierte Proteine bindet, wobei sich die Matrix in einem separaten Reaktionsgefäß außerhalb der Reaktionskammer befindet und über eine Zuleitung, eine Ableitung und, optional, eine Kreislaufpumpe mit der Reaktionskammer verbunden ist, wobei vor und nach der Matrix, bezogen auf die Strömungsrichtung der Reaktionslösung, Mittel zur Bestimmung der Proteinkonzentration eingerichtet sind.

19. Vorrichtung nach Anspruch 18, wobei die Mittel zur Bestimmung der Proteinkonzentration zur Bestimmung durch Absorption von Licht, insbesondere im UV-Bereich, eingerichtet sind.

20. Vorrichtung nach Anspruch 18 oder 19, wobei die Reaktionskammer einen durch eine semipermeable Membran abgetrennten Bereich enthält, in dem Reaktionslösung entnommen und/oder zugesetzt werden kann.

## Claims

1. A method for gene expression in a cell-free transcription/translation system, the reaction solution containing all components necessary for transcription/translation mechanism, amino acids, nucleotides, metabolic components, which provide energy and which are necessary for the synthesis,
wherein the proteins arising during the transcription/translation are immobilized on a matrix by that the reaction solution is conducted over the matrix disposed outside a reaction chamber serving for receiving the reaction solution in a separate reaction vessel passed by the reaction solution,
**characterized by** that the concentration of protein is determined before and behind the matrix referred to the flow direction of the reaction solution.

2. A method according to claim 1, **characterized by** that the matrix is arranged in a column.

3. A method according to claim 1 or 2, **characterized by** that the determination of the concentration of protein is performed by the absorption of light, in particular in the u.v. range.

4. A method according to one of claims 1 to 3, **characterized by** that the matrix specifically binds proteins by hydrophobic and/or hydrophilic or antigen/antibody or ionic interactions.

5. A method according to one of claims 1 to 4, **characterized by** that the matrix contains anion or cation exchange material or hydroxy-apatide.

6. A method according to one of claims 1 to 5, **characterized by** that the proteins are expressed as fusion proteins containing a fusion partner.

7. A method according to claim 6, **characterized by** that the fusion partners are located at the N terminal, C terminal or within the expressed protein.

8. A method according to claim 6 or 7, **characterized by** that a matrix is used, which specifically binds to the fusion partner.

9. A method according to one of claims 6 to 8, **characterized by** that the protein contains at the N terminal or C terminal several successive histidines, in particular 3 to 12, preferably 5 to 9, most preferably 6.

10. A method according to one of claims 1 to 9, **characterized by** that the matrix carries a metal chelate compound, in particular comprising bivalent metal ions, preferably bivalent copper and/or nickel ions.

11. A method according to one of claims 6 to 8, **characterized by** that the protein contains at the N terminal or C terminal glutathione S-transferase as a fusion partner.

12. A method according to one of claims 1 to 11, **characterized by** that glutathione is coupled to the matrix.

13. A method according to one of claims 6 to 8, **characterized by** that the protein contains an amino acid sequence permitting it to bind to streptavidin.

14. A method according to one of claims 1 to 13, **characterized by** that streptavidin is coupled to the matrix.

15. A method according to one of claims 1 to 14, **characterized by** that generated low-molecular metabolic products are extracted during the expression, and consumed necessary metabolic components are added to the reaction chamber in an amount such that the concentrations of the low-molecular metabolic products and the necessary metabolic components differ during the duration of the reaction by less than 80 %, preferably by less than 50 %, most preferably by less than 20 % of their a respective initial concentrations, each referred to moles.

16. A method according to claims 1 to 15, **characterized by** that the necessary metabolic components contain a substance of the group comprising "ATP, UTP, GTP and CTP, pyrophosphate, amino acids, and mixtures of these substances".

17. A method according to claims 1 to 16, **characterized by** that the necessary metabolic components are added via a semi-permeable membrane, and the low-molecular metabolic products are extracted via a semi-permeable membrane, preferably the same semi-permeable membrane, said semi-permeable membrane having an cut-off limit in the range from 500 to 20,000 Da, most preferably in the range from 3,000 to 5,000 Da.

18. A device for carrying-out a method according to one of claims 1 to 17, comprising a reaction chamber for receiving the reaction solution containing all the components necessary for the transcription/translation mechanism, amino acids, nucleotides, metabolic components, which provide energy and which are necessary for the synthesis, and a matrix specifically binding expressed proteins, wherein the matrix is located in a separate reaction vessel outside the reaction vessel and is connected by an inlet, an outlet and optionally a circular pump to the reaction vessel, and wherein there are provided means before and behind the matrix, referred to the flow direction of the reaction solution, for determining the protein concentration.

19. A device according to claim 18, wherein the means for determining the protein concentration are provided for the determination by absorption of light, in particular in the u.v. range.

20. A device according to claim 18 or 19, wherein the reaction chamber comprises a section separated by a semi-permeable membrane, and the reaction solution can be removed therefrom or added thereto.

## Revendications

1. Procédé pour l'expression génétique dans un système de transcription/traduction sans cellule, dans lequel la solution de réaction comprend tous les composants nécessaires pour l'appareil de transcription/traduction, des acides aminés, des nucléotides, des composants métaboliques, qui fournissent de l'énergie et qui sont nécessaires pour la synthèse,
dans lequel les protéines étant formées pendant la transcription/traduction sont immobilisées sur une matrice par ce que la solution de réaction est conduite sur la matrice disposée à l'extérieur d'une chambre de réaction servant à recevoir la solution de réaction dans un vaisseau de réaction séparé traversé par la solution de réaction,
**caractérisé par** ce que la concentration de protéine est déterminée en amont et en aval de la matrice en référence à la direction d'écoulement de la solution de réaction.

2. Procédé selon la revendication 1, **caractérisé par** ce que la matrice est arrangée dans une colonne.

3. Procédé selon la revendication 1 ou 2, **caractérisé par** ce que la détermination de la concentration de protéine se fait par l'absorption de lumière, en particulier dans la gamme u.v.

4. Procédé selon une des revendications 1 à 3, **caractérisé par** ce que la matrice lie spécifiquement des protéines par des interactions hydrophobiques et/ou hydrophiliques ou d'antigène/anticorps ou ioniques.

5. Procédé selon une des revendications 1 à 4, **caractérisé par** ce que la matrice comprend de la matière d'échange anionique ou cationique ou de l'hydroxyapatide.

6. Procédé selon une des revendications 1 à 5, **caractérisé par** ce que les protéines sont exprimées comme protéines de fusion comprenant un partenaire de fusion.

7. Procédé selon la revendication 6, **caractérisé par** ce que les partenaires de fusion se trouvent au N-terminal, C-terminal ou au-dedans de la protéine exprimée.

8. Procédé selon la revendication 6 ou 7, **caractérisé par** ce qu'une matrice est utilisée, qui lie spécifiquement le partenaire de fusion.

9. Procédé selon une des revendications 6 à 8, **caractérisé par** ce que la protéine comprend au N-terminal ou C-terminal plusieurs histidines successives, en particulier 3 à 12, de préférence 5 à 9, de préférence la plus haute 6.

10. Procédé selon une des revendications 1 à 9, **caractérisé par** ce que la matrice porte un composé de chélate de métal, en particulier comprenant des ions métalliques bivalents, de préférence des ions de cuivre et/ou de nickel bivalents.

11. Procédé selon une des revendications 6 à 8, **caractérisé par** ce que la protéine comprend au N-terminal ou C-terminal de la glutathione S-transférase comme partenaire de fusion.

12. Procédé selon une des revendications 1 à 11, **caractérisé par** ce que de la glutathione est couplée à la matrice.

13. Procédé selon une des revendications 6 à 8, **caractérisé par** ce que la protéine comprend une séquence d'acide aminé la permettant de se lier à la streptavidine.

14. Procédé selon une des revendications 1 à 13, **caractérisé par** ce que de la streptavidine est couplée à la matrice.

15. Procédé selon une des revendications 1 à 14, **caractérisé par** ce que des produits métaboliques générés à poids moléculaire bas sont extraits pendant l'expression, et des composants métaboliques nécessaires consommés sont ajoutés à la chambre de réaction dans une telle quantité, que les concentrations des produits métaboliques à poids moléculaire bas et les composants métaboliques nécessaires diffèrent pendant la durée de la réaction par moins que 80 %, de préférence par moins que 50 %, de préférence la plus haute par moins que 20 % des concentrations initiales respectives, chacune en référence à des moles.

16. Procédé selon une des revendications 1 à 15, **caractérisé par** ce que les composants métaboliques nécessaires comprennent une substance du groupe comprenant «l'ATP, l'UTP, le GTP et le CTP, le pyrophosphate, des acides aminés, et des mélanges des ces substances».

17. Procédé selon une des revendications 1 à 16, **caractérisé par** ce que les composants métaboliques nécessaires sont ajoutés par l'intermédiaire d'une membrane semi-perméable, et les produits métaboliques à poids moléculaire bas sont extraits par l'intermédiaire d'une membrane semi-perméable, de préférence la même membrane semi-perméable, cette membrane semi-perméable ayant une limite d'exclusion dans la gamme de 500 à 20.000 Da, de préférence la plus haute dans la gamme de 3.000 à 5.000 Da.

18. Dispositif pour la mise en oeuvre d'un procédé selon une des revendications 1 à 17, comprenant une chambre de réaction pour recevoir la solution de réaction comprenant tous les composants nécessaires de l'appareil de transcription/traduction, des acides aminés, des nucléotides, des composants métaboliques, qui fournissent de l'énergie et qui sont nécessaires pour la synthèse, et une matrice spécifiquement liant des protéines exprimées, dans lequel la matrice se trouve dans un vaisseau de réaction séparé à l'extérieur de la chambre de réaction et est liée par l'intermédiaire d'une entrée, d'une sortie et optionnellement d'une pompe circulaire à la chambre de réaction, et dans lequel sont prévus devant et derrière la matrice, en référence à la direction d'écoulement de la solution de réaction, des moyens pour déterminer la concentration de protéine.

19. Dispositif selon la revendication 18, dans lequel les moyens pour déterminer la concentration de protéine sont prévus pour la détermination par l'absorption de lumière, en particulier dans la gamme u.v.

20. Dispositif selon la revendication 18 ou 19, dans lequel la chambre de réaction comprend une section séparée par une membrane semi-perméable, et la solution de réaction peut être enlevée de celle-ci ou ajoutée à celle-ci.
